# EUROPÄISCHE PATENTSCHRIFT

(11) **EP 0 971 651 B1**
(45) Veröffentlichungstag und Bekanntmachung des Hinweises auf die Patenterteilung: **06.11.2002**
(21) Anmeldenummer: 98909489.1
(22) Anmeldetag: 02.03.1998
(51) Int. Cl.: A61F 2/36

(54) **HÜFTGELENKENDOPROTHESE**
HIP JOINT ENDOPROSTHESIS
ENDOPROTHESE DE L'ARTICULATION DE LA HANCHE

(30) Priorität: 26.03.1997 DE 29705500 U
(43) Veröffentlichungstag der Anmeldung: 19.01.2000
(73) Patentinhaber: Waldemar Link (GmbH & Co.), 22339 Hamburg (DE)
(72) Erfinder: KELLER, Arnold, D-23863 Kayhude (DE)
(74) Vertreter: Glawe, Delfs, Moll & Partner
(86) Internationale Anmeldenummer: EP9801158
(87) Internationale Veröffentlichungsnummer: WO98042279

(56) Entgegenhaltungen:
- EP-A- 0 477 113
- EP-A- 0 579 868
- EP-A- 0 666 069
- EP-A- 0 711 534
- WO-A-81/01510
- WO-A-87/00033
- FR-A- 2 429 010
- FR-A- 2 629 707
- FR-A- 2 636 837
- FR-A- 2 706 283
- GB-A- 2 069 340
- US-A- 5 413 610

## Beschreibung

In den Anfängen der Hüftgelenkprothetik gab es Versuche, lediglich den Gelenkkopf durch ein Implantat zu ersetzen, das von einem geraden Schaft gehalten ist, der vom Schenkelhals und der Spongiosa der sich daran anschließenden Epiphyse des Oberschenkelknochens gehalten ist. Es zeigte sich, daß diese Anordnung für die Übertragung der Kräfte auf den Oberschenkelknochen nicht ausreicht. Es werden deshalb meist Hüftprothesen verwendet, deren Schaft in die Diaphyse des Knochens reicht (FR-A-2 636 837, US-A-5,413,610, GB-A-2 069 340; EP-A-477 113, FR-A-2 706 283, EP-A-711 534, FR-A-2 629 707, FR-A-2 429 010). Um dafür hinreichenden Zugang zu finden, wird der Schenkelhals gänzlich reseziert. In den letzten Jahren wurden Vorschläge für Hüftprothesen geäußert, die die Erhaltung des Schenkelhalses erlauben. Die Resektion findet unmittelbar unterhalb des Gelenkkopfs statt (EP-A-666 069, Sp. 2, Z. 47; EP-A-579 868, Sp. 8, Z. 51; WO 87/00033, S. 6, Z. 19). Es ist eine kragenförmige Halsauflage vorgesehen, die sich an der Resektionsfläche des Schenkelhalses abstützt. Der Abstand der Auflagefläche der Halsauflage vom Zentrum des Gelenkkopfs der Prothese ist unveränderlich festgelegt, weil Variationen dank der Resektion unmittelbar unterhalb des Gelenkkopfs ausgeschlossen sind. Bei herkömmlich zu implantierenden Hüftprothesen ist es hingegen bekannt (FR-A-2 429 010, FR-A-2 629 707, US-A-5,413,610, FR-A-2 636 837), unterschiedliche Halslängen der Prothese zum Ausgleich unterschiedlicher Halslängen des zu ersetzenden Knochens vorrätig zu halten.

Die Erfindung geht von demjenigen Prothesentyp aus, der die Erhaltung des natürlichen Schenkelhalses anstrebt (EP-A-666 069). Ihre Aufgabe besteht darin, eine vielfältigere Anwendbarkeit zu erreichen, über die gegebenenfalls noch während der Operation entschieden werden kann.

Die erfindungsgemäße Lösung liegt in den Merkmalen des Anspruchs 1. Bei einer Hüftgelenkendoprothese (im nachfolgenden Hüftgelenkprothese) mit einem im Oberschenkelknochen zu verankernden, im wesentlichen gleichmäßig kreisbogenförmig gekrümmten Schaft mit einer Länge von nicht mehr als 150 mm und einem etwa gleichgerichtet an dessen oberes Ende anschließenden Hals, dessen Länge geringer ist als die Länge eines durchschnittlichen natürlichen Schenkelhalses und der einen Gelenkkopf trägt, ist eine Schaftform vorgesehen, deren geometrische Bedingungen in der Projektion auf die LM-Ebene (Lateral-Medial-Ebene) lauten:
- Die mediale Kontur im proximalen Abschnitt folgt einem Kreisbogen mit dem Radius K·7,2 und im distalen Abschnitt einem daran richtungsgleich anschließenden Kreisbogen mit dem Radius K·27;
- die laterale Kontur folgt im proximalen Abschnitt einem Kreisbogen mit dem Radius K·12,4 und im distalen Abschnitt einem richtungsgleich daran anschließenden Kreisbogen mit dem Radius K·21;
- an der Übergangsstelle vom proximalen zum distalen Abschnitt, die für die laterale und die mediale Kontur übereinstimmt, weisen die Kreisbögen, die diese beiden Konturen bestimmen, einen Abstand von 1,9 cm auf;
- die Radien der lateralen und medialen Kreisbögen schließen an der Übergangsstelle einen Winkel von 3,8° miteinander ein;
- K ist eine für jede Prothese einheitliche Konstante zwischen 0,8 und 1,1 cm;
- die Abweichungen der tatsächlichen Konturen von den genannten Kreisbögen sind nicht größer als 3 mm.

Die Konturen verschiedener Prothesengrößen sind miteinander verknüpft durch den Maßstabsfaktor K.

Diese Merkmalskombination gestattet es dem Arzt, je nach den Verhältnissen, die er bei der Operation vorfindet, den Schenkelhals ganz, teilweise oder nicht zu erhalten. Je nach Lage des Falles implantiert er die erfindungsgemäße Prothese in einer mehr oder weniger tiefen Position und hat es so in der Hand, den jeweils optimalen Kompromiß zwischen der Erhaltung von möglichst viel Knochenmaterial einerseits und der Schaffung einer stabilen Prothesenabstützung andererseits zu finden. Besonders bei jüngeren Patienten ist es wichtig, möglichst viel Knochensubstanz bei der ersten Prothesenimplantation für eine zu erwartende zweite oder gar dritte Prothesenversorgung zu erhalten.

Dank den genannten Merkmalen kann nämlich je nach Lage der Resektionsebene der Schaft parallel zu sich selbst mehr oder weniger weit auf entsprechend gekrümmtem Weg durch den Schenkelhals in den sich daran anschließenden Teil des Oberschenkelknochens eingeschoben werden. Die Spitze des Schaftes kann zwar bis in die Diaphyse hineinragen, aber dies ist erfindungsgemäß nur über eine begrenzte Strecke möglich; gleichwohl gelangt der Prothesenschaft in denjenigen Bereich des Oberschenkelknochens, der aufgrund der Dicke seiner Cortikalis gute Abstützung gewährt. Vorzugsweise ist die Schaftlänge noch beträchtlich kleiner als 150 mm, nämlich in der Größenordnung von 100 bis 140 mm, weiter vorzugsweise bis 130 mm. Die Länge ist von der Mitte des oberen Schaftendes (unter der Halsauflage, falls vorhanden) geradlinig bis zur Schaftspitze zu messen. Die Prothese sitzt also je nach dem Grad der Resektion des Schenkelhalses mehr oder weniger tief im Knochen. Damit der Gelenkkopf trotzdem und unabhängig vom Grad der Resektion des Schenkelhalses an gewünschter Stelle sitzt, sind erfindungsgemäß unterschiedliche Halslängen vorgesehen. Die verfügbaren Halslängen sind aus dem Grund kürzer als die durchschnittliche natürliche Schenkelhalslänge, da in der Regel mindestens ein Teil der Schenkelhalslänge stehen gelassen wird.

Während die bekannten Prothesen sämtlich eine vorbestimmte Resektionsebene verlangen, nämlich entweder unmittelbar unterhalb des Gelenkkopfs oder am distalen Ende des Schenkelhalses, ist dies bei der erfindungsgemäßen Prothese nicht der Fall. Vielmehr wird durch die gleichmäßige Krümmung des Schafts erreicht, daß er unterschiedlich tief implantiert werden kann, wobei die unterschiedliche Lage der Resektionsebene im Verhältnis zum Gelenkkopf durch Wahl einer geeigneten Halslänge ausgeglichen wird. Dies geschieht am einfachsten dadurch, daß der Hals mit einem Steckkonus zur Verbindung mit dem Gelenkkopf ausgerüstet ist und Gelenkköpfe mit unterschiedlicher Aufsteckstrecke zur Verfügung stehen.

Es kann zweckmäßig sein, wenn der Schaft in der Projektion auf die LM-Ebene zwischen zwei konzentrischen Kreisen liegt, deren Abstand der größten Dicke des Schafts gleicht.

In der Ansicht von lateral folgt die Kontur des Schafts dem Verlauf des Knochens. Insbesondere ist es zweckmäßig, auch die Anteversion des Halses getreu nachzubilden und entsprechend unterschiedliche Schaftausführungen für die beiden Körperseiten vorzusehen.

Vorzugsweise ist der Schaft ausschließlich auf seiner Vorderseite und seiner Rückseite mehrfach tief genutet, wobei der Verlauf der Nuten dem Krümmungsverlauf des Schafts parallel ist. Diese Nuten nehmen Knochenmaterial auf und tragen dadurch zur Abstützung des Schafts bei zementfreier Implantation bei. Dank ihrem zur Krümmung des Schafts parallelen Verlauf unterstützen sie durch ihre Führungseigenschaften das korrekte Einbringen der Prothese in den Knochen auf gekrümmter Bahn parallel zu sich selbst.

Der untere Abschnitt des Prothesenschafts, der etwa ein Drittel von dessen Gesamtlänge einnimmt, kann eine geringere Krümmung als der verbleibende obere Teil des Schafts aufweisen. Diese Krümmung kann um so geringer sein, je stärker die Dicke des Schafts zu seinem unteren Ende hin abnimmt.

Die Erfindung wird im folgenden näher unter Bezugnahme auf die Zeichnung erläutert, die ein vorteilhaftes Ausführungsbeispiel veranschaulicht. Darin zeigen:
- Fig. 1 bis 3: Ansichten der Prothese in der Projektion auf die LM-Ebene bei unterschiedlicher Lage der Resektionsfläche
- Fig. 4: eine Seitenansicht der Prothese in etwa natürlichem Maßstab,
- Fig: 5: Schaftquerschnitte an den Stellen A, B und C,
- Fig. 6: den Schaftquerschnitt an der Stelle D,
- Fig. 7: die geometrischen Bedingungen für die Umrißkontur des Prothesenschafts in der Projektion auf die LM-Ebene.

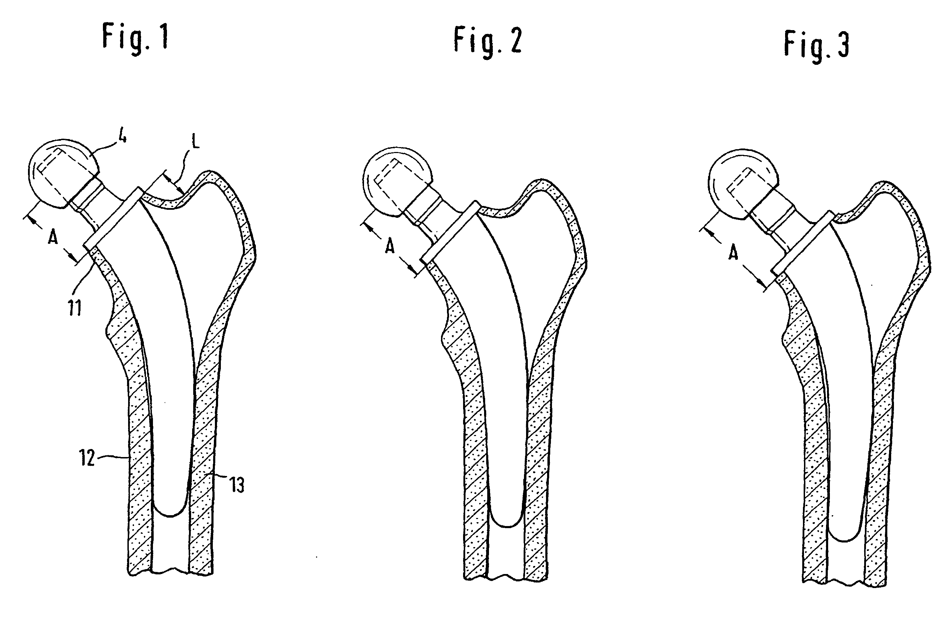

Die Prothese besteht aus einem Schaft 1, der oben durch eine Halsauflage 2 abgeschlossen wird, einem Hals 3 und einem Kopf 4, der eine Konusbohrung zum Aufsetzen auf den Konus 5 enthält, der die Prothese oben abschließt. Wie aus dem Vergleich der Fig. 1 bis 3 hervorgeht, haben die Köpfe 4 unterschiedlichen Bohrungsdurchmesser, der dazu führt, daß der Abstand A, der die Distanz zwischen der Unterfläche der Halsauflage 2 und dem Zentrum des Kopfs 4 angibt, unterschiedlich ausfällt.

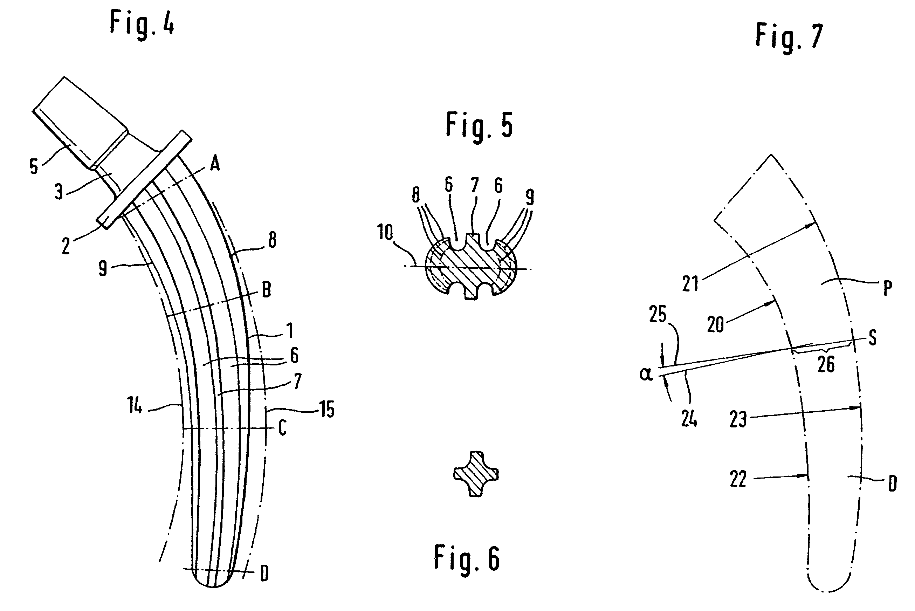

Der Schaft 1 der Prothese ist in der Projektion auf die LM-Ebene, in der er in der Zeichnung gezeigt ist, stark gekrümmt. Bei dem in Fig. 4 dargestellten Beispiel liegt die laterale und mediale Schaftbegrenzung zwischen zwei konzentrischen Kreisbögen 14 und 15, deren Abstand der maximalen Schaftdicke entspricht. Die Krümmung seiner Mittellinie folgt in den oberen beiden Dritteln (bis etwa zur Schnittebene C) einem Kreisbogen. Der Radius der strichpunktiert angegebenen Mittellinie liegt vorzugsweise zwischen 7 und 15 cm, weiter vorzugsweise zwischen 9 und 13 cm. Sie ist vorzugsweise zu den Kreisbögen 14, 15 in den oberen zwei Dritteln der Schaftlänge konzentrisch. Im unteren Abschnitt jenseits der Schnittlinie C ist der Schaft ebenfalls gekrümmt, aber mit größerem Krümmungsradius. Er kann hier auch gerade verlaufen, wenn er entsprechend kurz ist, nämlich zweckmäßigerweise nicht länger als die Hälfte der gleichmäßig gekrümmten Schaftlänge. Vorzugsweise ist er aber gleichfalls ein wenig gekrümmt und kann dann auch in bezug auf den oberen Schaftabschnitt entsprechend länger sein. Im dargestellten Fall beträgt seine längs der Mittellinie gemessene Länge etwa zwei Drittel des oberhalb der Schnittlinie C gleichmäßig gekrümmten Abschnitts.

Im Querschnitt ist der Schaft vorzugsweise länglich geformt mit der größeren Achse in LM-Richtung. Seine Ausdehnung in AP-Richtung im Mittelbereich ist im oberen Drittel der Schaftlänge (zwischen den Schnittlinien A und B) etwa konstant und verjüngt sich von da an etwa gleichmäßig bis zur Spitze. Dadurch stellt er in der Projektion auf eine Horizontalebene im oberen Drittel eine verhältnismäßig große Fläche zur Übertragung vertikaler Kräfte zur Verfügung. Die Querschnittsentwicklung erkennt man am besten in Fig. 5, in der mit ausgezogenen Linien die Querschnittsform in der Schnittebene A, mit gestrichelten Linien diejenige in der Schnittebene B und mit strichpunktierten Linien diejenige in der Schnittebene C gezeigt ist. Fig. 6 zeigt schließlich die Gestalt der Schnittebene D.

Man ersieht auch daraus, daß der Schaft genutet ist. Die Nuten 6 verlaufen parallel zur Schaftmittellinie und werden durch eine Rippe 7 getrennt, deren Höhe (entsprechend der Ausdehnung des Schafts in AP-Richtung) zwischen den Schnittlinien A und B unverändert bleibt und sich zur Schnittlinie C hin nur wenig verringert. Demgegenüber vermindert sich die Ausdehnung der lateralen und medialen Querschnittsbegrenzungen 8, 9 verhältnismäßig stärker. Der Nutgrund behält in den oberen zwei Dritteln der Schaftlänge etwa gleichen Abstand zur LM-Mittelebene 10 des Schafts. Aus diesen Merkmalen folgt, daß die Nutflanken in den oberen zwei Dritteln der Schaftlänge eine große Fläche zur Übertragung von medial gerichteten Kräften zur Verfügung stellen. Insbesondere im oberen Drittel wird außerdem eine große Fläche zur Übertragung in vertikaler Richtung zur Verfügung gestellt.

Die Schaftoberfläche ist zweckmäßigerweise im Sinne einer innigen Verbindung mit dem natürlichen Knochengewebe ausgebildet, beispielsweise aufgerauht oder mit einer Hydroxylapathit-Beschichtung versehen. Das gilt vornehmlich für die oberen zwei Drittel der Schaftlänge, während die Spitze ggf. glatt ausgebildet sein kann. Die Halsauflage dient, wie bekannt, zur zusätzlichen Übertragung von Kräften auf die Resektionsfläche. Die Nuten 6 können durch den Bereich der Halsauflage hindurchgeführt sein, um bei der Implantation das Stopfen von Knochenmaterial oder im Falle der Explantation die Einführung eines Lösewerkzeugs zu ermöglichen.

Die Gesamtkrümmung des Prothesenschafts entspricht etwa der gesamten Richtungsänderung zwischen dem Schenkelhals 11 und der Diaphyse 12. Die nahezu gleichmäßige Krümmung gestattet es, sie in eine entsprechend vorgearbeitete Knochenhöhlung einzuschieben. Dabei läßt die Schaftform große Freiheit bezüglich der Wahl der Resektionslänge L des Schenkelhalses, weil der Schaft mehr oder weniger weit eingeschoben werden kann, bevor sein unteres Ende durch Anschlag an der harten Knochenrinde 13 des diaphysären bzw. metaphysären Bereichs dem eine Grenze setzt.

Bei der Operation kann mit einer formgleichen Raspel vor dem Einsetzen des Prothesenschafts eine formgleiche Höhlung vorgearbeitet werden. Zweckmäßiger ist es im allgemeinen, zunächst mit einem dünneren Werkzeug einen engeren Kanal auszuarbeiten, in den der Prothesenschaft unter Verdrängung des verhältnismäßig weichen, spongiösen Knochenmaterials eingepreßt wird. Dadurch ergibt sich bei zementloser Implantation unmittelbar postoperativ ein festerer Sitz. Zusätzlich kann auch Knochenmaterial durch die Nutverläufe hindurch nachgestopft werden.

In der Projektion auf die AP-Ebene ist der Schaft zweckmäßigerweise entsprechend dem natürlichen Knochenverlauf gekrümmt. Im oberen Bereich weist er die Anteversion des Schenkelhalses auf.

Fig. 7 zeigt die Bestimmung der Schaftkontur der Prothese. Gezeigt sind strichpunktiert diejenigen Kreisbögen, aus denen die Kontur idealerweise zusammengesetzt ist. Zwar wird man zweckmäßigerweise unterschiedliche Größenausfertigungen der Prothese innerhalb einer Typenserie vorsehen. Da die Schäfte einander aber im wesentlichen ähnlich sind, kann die Idealform für sie alle einheitlich dargestellt und über einen Umrechnungsfaktor K angepaßt werden, der das Größenverhältnis der jeweils betrachteten Prothese im Verhältnis zur Idealform angibt und dementsprechend im vorliegenden Fall zwischen 0,8 und 1,1 liegen soll.

Der proximale Abschnitt P wird auf der medialen Seite von einem Kreisbogen begrenzt, dessen Radius 20 zweckmäßigerweise zwischen 6 und 8 cm liegt. Er beträgt im dargestellten Beispiel 7,2 cm. Der Radius 21 des lateralen Kreisbogens liegt zweckmäßigerweise zwischen 11 und 14 cm, weiter vorzugsweise beträgt er gemäß dem dargestellten Beispiel 12,4 cm. An der Übergangsstelle S endet der proximale Abschnitt und geht in den distalen Abschnitt D über. An dieser Stelle gehen die Kreisbögen mit den Radien 20, 21 richtungsgleich in Kreisbögen mit den Radien 22, 23 über. Das bedeutet, daß die Radien 20 und 22 an der Übergangsstelle in Gestalt des Radius 24 zusammenfallen, während die Radien 21 und 23 in Gestalt des Radius 25 zusammenfallen. Die Radien 24 und 25 weichen (entsprechend der Keilform des Schafts) im Winkel α voneinander ab. Die Winkelabweichung beträgt zweckmäßigerweise 3 bis 5° und im dargestellten Beispiel 3,8°.

Der Radius 22 auf der lateralen Seite des distalen Abschnitts liegt zweckmäßigerweise zwischen 22 und 35 cm. Er beträgt im dargestellten Beispiel 27 cm. Der Radius 23 liegt zweckmäßigerweise zwischen 18 und 25 cm. Er beträgt im dargestellten Beispiel 21 cm. Der Abstand zwischen der lateralen und der medialen Kontur wird am leichtesten an der Übergangsstelle S bestimmt. Er liegt zweckmäßigerweise zwischen 1,7 und 2,1 cm, vorzugsweise zwischen 1,8 und 2,0 cm. Er beträgt im dargestellten Beispiel 1,9 cm. In der Praxis sind kleine Abweichungen von der Idealkontur teils unvermeidlich, teils auch zur Anpassung an die jeweiligen Erfordernisse gewollt. Sie sollen aber nicht über 3 mm, vorzugsweise nicht über 2 mm liegen. Wenn an einer Stelle die wahre Kontur von der Idealkontur erheblich in einer Richtung abweicht, soll sie an der gegenüberliegenden Stelle in derselben Richtung von der Idealkontur abweichen. Wie oben bereits gesagt wurde, sind die angegebenen Abmessungen gemäß dem frei wählbaren Faktor K entsprechend der gewünschten Größenstufe der Prothese zu korrigieren.

Die Länge der Prothesenschäfte ist innerhalb einer und zwischen verschiedenen Prothesengrößen unterschiedlich. Die Länge des Prothesenschafts, geradlinig gemessen von der Mitte seines Ansatzes an der Halsauflage bis zur Spitze, liegt zweckmäßigerweise zwischen 10 und 13 cm. Zweckmäßige Größen sind beispielsweise 105, 115 und 125 mm Schaftlänge. Der Bogen der oberen zwei Drittel der Schaftlänge erstreckt sich zweckmäßigerweise über etwa 35 bis 45 Grad, die Krümmung im unteren Drittel über 2 bis 10, vorzugsweise etwa 5 Grad. Der Hals geht zwar etwa gleichgerichtet aus dem oberen Schaftende hervor, kann aber mit diesem einen spitzen Winkel von vorzugsweise 0 bis 20 Grad einschließen. Die Summe dieser Winkel bildet den Komplementärwinkel zu dem sogenannten CCD-Winkel, der zweckmäßigerweise zwischen 110 und 130 Grad liegt.

## Patentansprüche

1. Hüftgelenkendoprothese mit einem im Oberschenkelknochen zu verankernden, im wesentlichen gleichmäßig kreisbogenförmig gekrümmten Schaft (1) mit einer Länge von nicht mehr als 150 mm und einem etwa gleichgerichtet an dessen oberes Ende anschließenden Hals (3), dessen Länge geringer ist als die Länge eines durchschnittlichen natürlichen Schenkelhalses und der einen Gelenkkopf (4) trägt, **dadurch gekennzeichnet, daß** die Prothese mit unterschiedlichen Halslängen an einem Schaft verfügbar ist, dessen mediale Kontur im proximalen Abschnitt (P) einem Kreisbogen (20) mit der Radiuslänge K·(6 bis 8) und im distalen Abschnitt (D) einem daran richtungsgleich anschließenden Kreisbogen (22) mit der Radiuslänge K·(22 bis 35) folgt und dessen laterale Kontur im proximalen Abschnitt (P) einem Kreisbogen (21) mit der Radiuslänge K·(11 bis 14) und im distalen Abschnitt (D) einem richtungsgleich daran anschließenden Kreisbogen (23) mit der Radiuslänge K·(18 bis 25) folgt, wobei die die beiden Konturen bestimmenden Kreisbögen an der Übergangsstelle (S) vom proximalen zum distalen Abschnitt einen Abstand (26) von 1,7 bis 2,1 cm aufweisen und ihre Radien einen Winkel (α) von 3 bis 5° miteinander bilden, wobei K eine für jede Prothese einheitliche Konstante zwischen 0,8 und 1,1 cm ist und die Abweichungen der Konturen von den genannten Kreisbögen nicht größer als 3 mm sind.

2. Prothese nach Anspruch 1, **dadurch gekennzeichnet, daß** der Schaft in der Projektion auf die LM-Ebene zwischen zwei konzentrischen Kreisbögen (14, 15) liegt, deren Abstand der größten Dicke des Schafts gleicht.

3. Prothese nach Anspruch 2, **dadurch gekennzeichnet, daß** die Kreisbögen (14, 15) konzentrisch zu der etwa einem Kreisbogen folgenden Mittellinie des Schafts in deren oberen Längenhälfte sind.

4. Prothese nach einem der Ansprüche 1 bis 3, **dadurch gekennzeichnet, daß** der Schaft (1) auf seiner Vorderseite und seiner Rückseite jeweils mehrere tiefe Nuten (6) aufweist, deren Verlauf demjenigen der Schaftmittellinie parallel ist.

5. Prothese nach einem der Ansprüche 1 bis 4, **dadurch gekennzeichnet, daß** die unterschiedlichen Halslängen durch die Kombination von übereinstimmenden Prothesenteilen mit konischem Halsende einerseits und mit Gelenkköpfen unterschiedlicher Konusbohrung andererseits verwirklicht sind.

## Claims

1. Hip-joint endoprosthesis with a stem (1) which is to be anchored in the femur, is curved substantially uniformly in a circular arc and has a length of not more than 150 mm, and with a neck (3) which adjoins the upper end of the stem approximately in the same direction and whose length is smaller than the length of an average natural femoral neck and supports a joint head (4), **characterized in that** the prosthesis is available with different neck lengths on a stem whose medial contour in the proximal section (P) follows a circular arc (20) with radius K·(6 to 8) and in the distal section (D) follows a co-directional contiguous circular arc (22) with radius K·(22 to 35); and whose lateral contour in the proximal section (P) follows a circular arc (21) with radius K·(11 to 14) and in the distal section (D) follows a co-directional contiguous circular arc (23) with radius K·(18 to 25); the circular arcs determining the two contours having, at the crossover point (S) from the proximal to the distal section of a distance (26) of 1.7 to 2.1 cm, and their radii forming an angle of 3 to 5° to one another; K being a constant of between 0.8 and 1.1 cm uniform for each prosthesis, and the deviations of the contours from the said circular arcs being not greater than 3 mm.

2. Prosthesis according to Claim 1, **characterized in that** the stem in the projection on the LM plane lies between two concentric circular arcs (14, 15), whose spacing is equal to the greatest thickness of the stem.

3. Prosthesis according to Claim 2, **characterized in that** the circular arcs (14, 15) are concentric to the centre line, approximately following a circular arc, of the stem in the upper half of its length.

4. Prosthesis according to one of Claims 1 to 3, **characterized in that** the stem (1) has, on its front and on its rear, in each case a plurality of deep grooves (6) whose course is parallel to that of the centre line of the stem.

5. Prosthesis according to one of Claims 1 to 4, **characterized in that** the different neck lengths are obtained by the combination of corresponding prosthesis parts with conical neck end, on the one hand, and with joint heads of a different conical bore, on the other hand.

## Revendications

1. Endoprothèse de la hanche comprenant une tige (1), recourbée pour l'essentiel en arc de cercle symétrique, destinée à être fixée dans le fémur, d'une longueur ne dépassant pas 150 mm et munie d'un col (3) contigu, convergeant approximativement en direction de son extrémité supérieure, d'une longueur inférieure à la longueur d'un col du fémur naturel moyen et présentant une tête d'articulation (4), **caractérisée en ce que** la prothèse est disponible avec différentes longueurs de col au niveau de la tige dont le contour médial, dans la section proximale (P), forme un arc de cercle (20) avec la longueur de rayon K • (de 6 à 8) et, dans la section distale (D), un arc de cercle (22), s'y rattachant dans la même direction, avec la longueur de rayon K • (de 22 à 35) et dont le contour latéral, dans la section proximale (P), forme un arc de cercle (21) avec la longueur de rayon K • (de 11 à 14) et, dans la section distale (D), un arc de cercle (23), s'y rattachant dans la même direction, avec la longueur de rayon K • (de 18 à 25), les arcs de cercle déterminant les deux contours, au niveau de la jonction (S) de la section proximale et de la section distale, présentant un écart (26) de 1,7 à 2,1 cm et leurs rayons formant ensemble un angle (α) de 3 à 5°, K étant une constante uniforme pour chaque prothèse, se situant entre 0,8 et 1,1 cm, et les écarts entre les contours et les arcs de cercle mentionnés ne dépassant pas 3 mm.

2. Prothèse selon la revendication 1, **caractérisée en ce que** la tige, dans la projection sur le plan LM, se situe entre les deux arcs de cercle concentriques (14, 15) dont l'écart est égal à l'épaisseur maximale de la tige.

3. Prothèse selon la revendication 2, **caractérisée en ce que** les deux arcs de cercle (14, 15) sont positionnés de manière concentrique par rapport à la ligne médiane de la tige formant approximativement un arc de cercle dans la moitié supérieure de leur longueur.

4. Prothèse selon l'une des revendications 1 à 3 **caractérisée en ce que** la tige (1) présente sur sa face avant et sa face arrière diverses rainures profondes (6) dont le cours est parallèle à celui de la ligne médiane de la tige.

5. Prothèse selon l'une des revendications 1 à 4, **caractérisée en ce que** les différentes longueurs du col sont obtenues par la combinaison de pièces de prothèse concordantes avec, d'une part, une extrémité du col conique et, d'autre part, des têtes d'articulation munies d'une cavité conique différente.
